# EUROPEAN PATENT SPECIFICATION

(11) **EP 4 183 376 B1**
(45) Date of publication and mention of the grant of the patent: **02.12.2026**
(21) Application number: 21833784.8
(22) Date of filing: 13.08.2021
(51) Int. Cl.: A61F 2/90

(54) **VASCULAR STENT**
GEFÄSSSTENT
ENDOPROTHÈSE VASCULAIRE

(30) Priority: 30.06.2020 CN 202010615047
(43) Date of publication of application: 24.05.2023
(73) Proprietor: MicroPort NeuroTech (Shanghai) Co., Ltd., Shanghai 201318 (CN)
(72) Inventor: ZHANG, Han, Shanghai 201318 (CN); YU, Peng, Shanghai 201318 (CN); TIAN, Hao, Shanghai 201318 (CN); WANG, Yiqun Bruce, Shanghai 201318 (CN); LIU, Jianmin, Shanghai 201318 (CN)
(74) Representative: Marks & Clerk LLP
(86) International application number: PCT/CN2021/112630
(87) International publication number: WO 2022/002281

(56) References cited:
- EP-A2- 2 762 622
- CA-A1- 3 090 382
- CN-A- 102 429 750
- CN-A- 105 125 326
- CN-A- 113 143 555
- CN-U- 205 515 056
- CN-U- 212 234 823
- US-A- 5 718 159
- US-A1- 2003 009 215
- US-A1- 2020 046 526
- US-A1- 2020 046 527

## Description

### TECHNICAL FIELD

The present invention relates to the field of medical devices and, in particular, to vascular stents.

### BACKGROUND

Most intracranial aneurysms are visualized as abnormal ballooning in the walls of cerebral arteries and are the No. 1 cause of subarachnoid hemorrhage. Among cerebrovascular accidents, intracranial aneurysms rank third after cerebral thrombosis and hypertensive cerebral hemorrhage. They may occur at any age, especially to in middle-aged and elderly women between the ages of 40 and 60. Although the etiology of intracranial aneurysms remains unknown, most scholars believe that they develop from congenital local defects in intracranial artery walls and increased intraluminal pressures, and their occurrence and progression are related to hypertension, cerebral arteriosclerosis and vasculitis. Intracranial aneurysms often occur in the cerebral arterial circle (circle of Willis), and 80% of them occur in the anterior part of the cerebral arterial circle.

Currently, the treatment of intracranial aneurysms relies mainly on surgical clipping and interventional endovascular aneurysm embolization. The surgical approach is highly invasive and associated with many side effects, causing significant pain to patients. Studies have shown that 85% of narrow-necked aneurysms can be completely occluded, whereas only 15% of wide-necked aneurysms can be completely occluded. For endovascular treatment of complex giant aneurysms, the failure of sufficiently dense packing and aneurysmal recurrence are of the greatest concern. The concept of endovascular reconstruction of parent arteries by the application of endovascular stents or something like that was proposed in the late 1980s and put into clinical use 15 years ago. Fluoroscopic visualization of existing braided stents is usually enabled by radiopaque features attached to the stent body, radiopaque wires braided in the stent body, and so forth. However, these stents are only partially visible. When such a stent is to be deployed in a curved blood vessel, due to its braid pitch, it would be difficult to determine its success in adhering to the blood vessel wall only based on a few fluoroscopically visible wires. In the case of a tapered blood vessel, as a stent's braid pitch will vary to adapt the stent to the varying diameter of the blood vessel, a physician would not be able to confidently determine whether the stent has achieved full expansion.

EP2762622 discloses a braided mesh for a medical implant or instrument. The mesh has wire strands intertwined with one another. The material wires comprise a core wire made from X-ray-viewable material and a cover made from shape memory material. CA3090382 discloses vascular expandable devices including a generally tubular sidewall formed of a plurality of braided strands. The device can have a compressed state for delivery and an expanded state. US5718159 discloses a process for manufacturing a prosthesis for intraluminal implantation having a flexible tubular three-dimensionally braided structure of metal or polymeric monofilaments, and polymeric multifilament yarns.

### SUMMARY OF THE INVENTION

The problem sought to be solved by the present invention is to provide vascular stents, each of which is wholly fluoroscopically visible such that its boundaries can be seen during a surgical procedure, allowing a physician to clearly determine whether it has adhered to a blood vessel wall. Moreover, they can provide strong support.

In order to solve the above problem, the present invention provides a vascular stent in the form of a self-expanding braided tubular structure constructed from at least one first braid wire and a composite braid wire, which are interwoven with each other. Each first braid wire includes a first wire core and a cladding jacket covering the first wire core, and the composite braid wire consists of at least one first braid wire and at least one second braid wire, wherein each second braid wire includes a second wire core and an opaque filament wound on the second wire core.

Preferably, the vascular stent is a self-expanding braided tubular structure constructed from the at least one first braid wire and the composite braid wire, which are spiral and interwoven with each other.

Preferably, the vascular stent is a self-expanding braided tubular structure constructed from the at least one first braid wire and the composite braid wire, which are interwoven with each other so as to spiral respectively clockwise and counterclockwise from opposing ends of the vascular stent.

Preferably, the cladding jacket is made of an elastic biomaterial and the first wire core of a radiopaque material. Moreover, the material of the first wire core has a linear attenuation coefficient higher than a linear attenuation coefficient of the material of the cladding jacket.

Preferably, the radiopaque material is one of platinum, iridium, gold, silver and tantalum, or an alloy thereof, and the elastic biomaterial is one or more of a nickel-titanium alloy, nitinol, stainless steel, a cobalt-chromium alloy and a nickel-cobalt alloy.

Preferably, the first braid wire has a cross-section shape of circular, square, elliptic or trapezoidal.

Preferably, a cross-section area of the first wire core accounts for 10-40% of a total cross-section area of the first braid wire.

Preferably, among all the materials in the first and second braid wires, a greatest linear attenuation coefficient does not exceed 25 times a lowest linear attenuation coefficient.

Preferably, a linear attenuation coefficient of the opaque filament does not exceed 25 times of a linear attenuation coefficient of the first wire core.

Preferably, the second braid wire has a cross-section shape of circular, square, elliptic or trapezoidal.

Preferably, the opaque filament is one of platinum, iridium, gold, silver and tantalum, or an alloy thereof, and the second wire core is a nickel-titanium alloy or one first braid wire.

Preferably, an axial distance between any two adjacent coils of the opaque filament wound on the second wire core is 1.0-1.5 times a diameter of the opaque filament.

Preferably, a number of the first braid wires is 24-96 and a number of the second braid wires is 2-6.

Preferably, the first braid wire and/or the second braid wire is/are surface coated by spraying or dipping with a drug coating, an antithrombotic coating and/or a hydrophilic coating.

Preferably, the self-expanding braided tubular structure has a first diameter less than 0.74 mm in a collapsed configuration and a second diameter in the range of 1.5 mm to 7 mm in an expanded configuration. Additionally, it expands from the first diameter to the second diameter under the action of a force not less than 0.01-1 N**.**

Preferably, each second braid wire has a cross-section diameter not greater than 90 µm, and each first braid wire has a cross-section diameter not greater than 50 µm.

Preferably, the first diameter of the self-expanding braided tubular structure in the collapsed configuration is less than 0.6858 mm, wherein the cross-section diameter of each second braid wire is not greater than 80 µm and the cross-section diameter of each first braid wire is not greater than 45 µm.

Preferably, the first diameter of the self-expanding braided tubular structure in the collapsed configuration is less than 0.5334 mm, wherein the cross-section diameter of each second braid wire is not greater than 50 µm and the cross-section diameter of each first braid wire is not greater than 35 µm.

Preferably, the first diameter of the self-expanding braided tubular structure in the collapsed configuration is less than 0.4826 mm, wherein the cross-section diameter of each second braid wire is not greater than 30 µm and the cross-section diameter of each first braid wire is not greater than 30 µm.

Preferably, in the expanded configuration, the self-expanding braided tubular structure has a first section, an intermediate section and a second section, which are sequentially joined together. Moreover, the intermediate section of the self-expanding braided tubular structure has a constant outer diameter, and each of the first section and the second section of the self-expanding braided tubular structure has an outer diameter gradually increasing from the intermediate section toward a corresponding end opening of the self-expanding braided tubular structure.

Preferably, the intermediate section has an axial length in the range of 2 mm to 60 mm, and each of the first section and the second section has an axial length in the range of 0.5 mm to 3 mm. In addition, wire segments in the first section or the second section, the outer diameter of which gradually increases from the intermediate section toward the corresponding end opening of the self-expanding braided tubular structure extend at an angle of 10-60° with respect to a center axis of the self-expanding braided tubular structure.

Preferably, the first section and the second section of the self-expanding braided tubular structure are flared in shape, in the shape of truncated cones, or tapered, or have star-shaped cross-sections.

Preferably, the self-expanding braided tubular structure has an axial length of 2-70 mm, and the interwoven two sets of braid wires form an angle of 100-140° axially and form an angle of 40-80° circumferentially.

Compared with the prior art, the present invention has the following benefits. The vascular stent of the present invention is provided in the form of a self-expanding braided tubular structure, in which each first braid wire contains both a radiopaque material and a shape memory alloy. As such, the entire stent, as well as each braid wire, is fluoroscopically visible and has shape memory properties, enabling self-expansion to a predetermined size. The opaque cores are visible on DSA such that boundaries of the vascular stent can be clearly identified during a surgical procedure, and a physician can clearly determine whether the vascular stent has adhered to the wall of an intended blood vessel. In particular, the second braid wires added are more radiopaque and thus more fluoroscopically visible under X-ray than other braid wires. Moreover, they are arranged at circumferentially opposing locations. In this way, it can be determined whether there is inward warpage at a leading end of the expanded vascular stent simply by checking a diameter at the end through measuring the distance(s) between the circumferential locations.

### BRIEF DESCRIPTION OF THE DRAWINGS

Fig. 1 shows a schematic overview of the structure of a vascular stent according to an embodiment of the present invention.
Fig. 2 shows a schematic cross-section view of a first braid wire according to an embodiment of the present invention.
Fig. 3 shows a schematic cross-section view of a second braid wire according to an embodiment of the present invention.

In these figures,
1-composite braid wire; 11-first braid wire; 12-second braid wire; 111-first wire core; 112-cladding jacket; 121-second wire core; 122-opaque filament; 21-intermediate section; 22-first section; 23-second section; 3-mesh opening.

### DETAILED DESCRIPTION

The present invention will be further described below with reference to the accompanying drawings and specific embodiments.

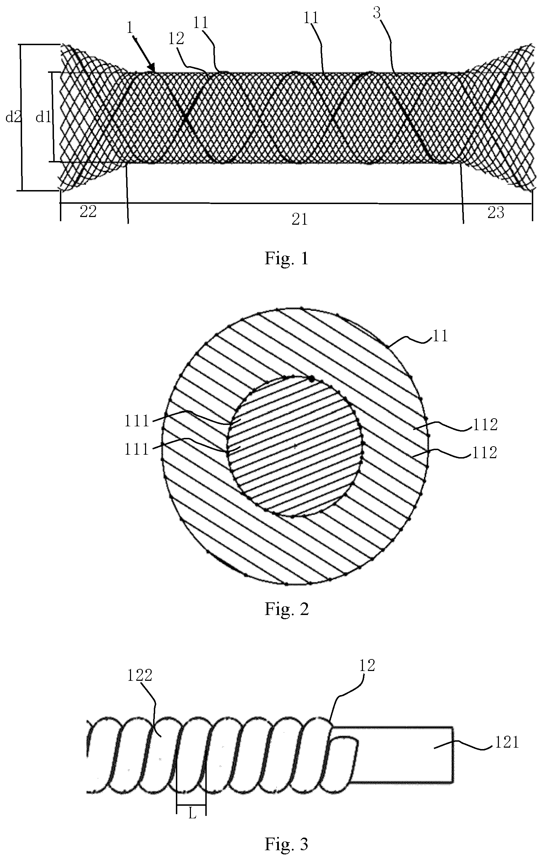

Fig. 1 shows a schematic overview of the structure of a vascular stent according to an embodiment of the present invention. Fig. 2 shows a schematic cross-section view of a first braid wire according to an embodiment of the present invention.

Referring to Figs. 1 and 2, a vascular stent is provided in an embodiment of the present invention in the form of a self-expanding braided tubular structure constructed from interwoven first and second components, or from interwoven second components. In a preferred embodiment, the vascular stent is a self-expanding braided tubular structure constructed from interwoven spiral first and second components. In a more preferred embodiment, the vascular stent is a self-expanding braided tubular structure constructed from interwoven first and second components spiraling from opposing ends of the vascular stent clockwise and counterclockwise, respectively. The first components include at least one first braid wire 11, each one first braid wire 11 includes a first wire core 111 and a cladding jacket 112 covering the first wire core 111. The first wire core 111 is made of a radiopaque material, which may be platinum, iridium, gold, silver, tantalum, an alloy thereof or the like. The cladding jacket 112 is made of an elastic biomaterial, which may be one or more of a nickel-titanium alloy, nitinol, stainless steel, a cobalt-chromium alloy and a nickel-cobalt alloy. The material of the first wire core 111 has a linear attenuation coefficient higher than that of the cladding jacket 112. Therefore, the first wire core 111 is more radiopaque. The first braid wire 11 may have a circular, square, elliptic, trapezoidal or an otherwise shaped cross-section, with a circular cross-section being more preferred. A cross-section area of the first wire core 111 accounts for 10-40% of a total cross-section area of the first braid wire 11. Preferably, the cross-section area of the first wire core 111 accounts for 30% of the total cross-section area of the first braid wire 11.

Referring to Fig. 3, the second components include at least one first braid wire 11 and at least one second braid wire 12. Each second braid wire 12 includes a second wire core 121 and an opaque filament 122 wound on the second wire core 121. The opaque filament 122 may be made of a radiopaque material such as platinum, iridium, gold, silver, tantalum or an alloy thereof. The second wire core 121 is preferred to be made of a nickel-titanium alloy. Alternatively, the second wire core 121 may be implemented as one first braid wire 11. Further, the composite braid wires 1 each consisting of a combination of one second braid wire 12 and one first braid wire 11 may be spiraled and interwoven with the first components. The opaque filament 122 has a linear attenuation coefficient higher than that of the second wire core 121. Each material has a linear attenuation coefficient (or linear absorption coefficient) µ₁, which is the product of a mass attenuation coefficient µm and the density ρ of the material. A higher linear attenuation coefficient means greater radiopaqueness. Preferably, among the materials in the first and second braid wire 11, 12, the highest linear attenuation coefficient is not higher than 25 times the lowest linear attenuation coefficient. Further, the linear attenuation coefficient of the opaque filament 122 does not exceed 25 times the linear attenuation coefficient of the first wire core 111.

The second braid wire 12 may have a circular, square, elliptic, trapezoidal or an otherwise shaped cross-section, with a circular cross-section being more preferred. Referring to Fig. 3, preferably, an axial distance L between any two adjacent active coils of the opaque filament 122 wound on the second wire core 121 is 1.0-1.5 times a diameter of the opaque filament 122. More preferably, the axial distance L between any two adjacent active coils of the opaque filament 122 is 1.0-1.2 times the diameter of the opaque filament 122.

Additionally, in the self-expanding braided tubular structure, the number of second braid wire 12 is less than the number of first braid wires 11. The number of first braid wires 11 is preferred to be between 24 and 96, and the number of second braid wires 12 is preferred to be between 2 and 6. More preferably, the number of first braid wires 11 is preferred to be between 44 and 62, and the number of second braid wires 12 is preferred to be between 2 and 4. Preferably, in an expanded configuration of the self-expanding braided tubular structure, the first braid wires 11 and the second braid wires 12 are evenly spaced circumferentially.

Further, the first braid wires 11 and the second braid wires 12 may be surface-coated, by spraying or dipping, with a drug coating, an antithrombotic coating, a hydrophilic coating and/or the like, as required. In this way, the vascular stent can find better use and have improved performance.

The self-expanding braided tubular structure has a first diameter when in a collapsed configuration and a second diameter when in the expanded configuration. The self-expanding braided tubular structure may be delivered by a delivery catheter while having the first diameter to a target diseased site, where it may expand to have the second diameter. The first diameter may be less than 0.74 mm, and accordingly, the delivery catheter may have an inner diameter of 0.029 inches or less, wherein 1 inch corresponds to 25.4 millimetres. The second diameter may range from 1.5 mm to 7 mm. Since the first and second braid wires both contain elastic materials, the expansion of the self-expanding braided tubular structure from the first diameter to the second diameter may occur under the action of a force in the range of 0.01 N to 1 N, and the vascular stent in the expanded configuration can provide strong support. Therefore, since each braid wire in the vascular stent contains both a radiopaque material and an elastic material, the vascular stent, as well as each braid wire, is wholly fluoroscopically visible and exhibits shape memory properties enabling self-expansion to a predetermined size. The radiopaque first wire cores 111 are visible on digital subtraction angiography (DSA), enabling identifiable boundaries of the vascular stent during a surgical procedure and allowing a physician to clearly determine whether the vascular stent has adhered to the wall of an intended blood vessel.

In the self-expanding braided tubular structure, the spiral first braid wires 11 and composite braid wires 1 are so interwoven that mesh openings 3 are formed having an axial size preferably of 0.1 mm to 1 mm. More preferably, the axial size ranges from 0.1 mm to 0.5 mm. The size of the mesh openings may depend on the number and a braid angle of the braid wires. Preferably, the self-expanding braided tubular structure has an axial length of 2-70 mm, and the two sets of braid wires are interwoven at an angle of 100-140° axially and of 40-80° circumferentially. In some embodiments, the two sets of braid wires are interwoven at an angle of 110-130° axially and of 50-70° circumferentially.

The self-expanding braided tubular structure may have a metal coverage of 20-40%, with 25-35% being more preferred. Here, the metal coverage refers to a ratio of a surface area of the metal wires in the self-expanding braided tubular structure to a total surface area of the self-expanding braided tubular structure.

In the expanded configuration, the self-expanding braided tubular structure includes a first section 22, an intermediate section 21 and a second section 23, which are sequentially joined together. In the self-expanding braided tubular structure, the intermediate section 21 maintains a constant outer diameter, while each of the first section 22 and the second section 23 has an outer diameter gradually increasing from the intermediate section 21 toward a corresponding end opening of the self-expanding braided tubular structure. Thus, the outer diameters d2 of the first section 22 and the second section 23 at the end openings are greater than the diameter d1 of the intermediate section 21. The first section 22 and the second section 23 may be, but are not necessarily, both flared in shape, as shown in Fig. 1. Alternatively, these sections may be in the shape of truncated cones, tapered or otherwise shaped, or the first section 22 and the second section 23 may have star-shaped cross-sections, without departing from the scope of the present invention. Preferably, both the first section 22 and the second section 23 have an axial length of 0.5-3 mm, and wire segments in the first section 22 or the second section 23 spiral at an angle of 10-60° with respect to a center axis of the self-expanding braided tubular structure. Preferably, the angle α at which the wire segments in the first section 22 and the second section 23 spiral with respect to the center axis of the self-expanding braided tubular structure is in the range of 30-60°. Preferably, the angle α at which the wire segments in the first section 22 and the second section 23 spiral with respect to the center axis of the self-expanding braided tubular structure is in the range of 30-45°.

During use, the self-expanding braided tubular structure can be collapsed to the first diameter. In one embodiment, it may be delivered to the site of an aneurysm using a 0.029-inch catheter, wherein a cross-section diameter of the second braid wires 12 is not greater than 90 µm, and a cross-section diameter of the first braid wire 11 is not greater than 50 µm.

In another embodiment, it may be delivered to the site of an aneurysm using a 0.027-inch catheter. That is, the diameter of the self-expanding braided tubular structure in the collapsed configuration is less than 0.027 inches. In this case, the cross-section diameter of the second braid wires 12 is not greater than 80 µm, and the cross-section diameter of the first braid wire 11 is not greater than 45 µm.

In other embodiments, the self-expanding braided tubular structure may be delivered to the site of an aneurysm using a 0.021-inch catheter. That is, its diameter in the collapsed configuration is less than 0.021 inches. In this case, the cross-section diameter of the second braid wires 12 is not greater than 50 µm, and the cross-section diameter of the first braid wire 11 is not greater than 35 µm.

In other embodiments, the self-expanding braided tubular structure may be delivered to the site of an aneurysm using a 0.017-inch catheter. That is, its diameter in the collapsed configuration is less than 0.017 inches. In this case, the cross-section diameter of the second braid wires 12 is not greater than 30 µm, and the cross-section diameter of the first braid wire 11 is not greater than 30 µm.

### Embodiment 1

Referring to Figs. 1, 2 and 3, a vascular stent according to an embodiment is provided in the form of a self-expanding braided tubular structure constructed from interwoven spiral first and second components. The first components are first braid wires 11, and the second components are composite braid wires 1 consisting of combined first braid wires 11 and second braid wires 12. Each first braid wire 11 includes a first wire core 111 and a cladding jacket 112 covering the first wire core 111. The first wire core 111 is made of platinum, and the cladding jacket 112 is made of a nickel-titanium alloy. Each second braid wire 12 includes a second wire core 121 and an opaque filament 122 wound on the second wire core 121. The opaque filament 122 is a platinum filament, and the second wire core 121 is made of a nickel-titanium alloy. In the first braid wires 11, the first wire cores 111 have a linear attenuation coefficient of 5148 cm⁻¹, and the cladding jackets 112 have a linear attenuation coefficient of 258 cm⁻¹. In the second braid wires 12, the opaque filaments 122 have a linear attenuation coefficient of 5148 cm⁻¹, and the second wire cores 121 have a linear attenuation coefficient of 258 cm⁻¹. Among the four materials from which the self-expanding braided tubular structure is fabricated, the ratio of the highest linear attenuation coefficient to the lowest linear attenuation coefficient is about 20.

The second braid wires 12 have a circular cross-section. Referring to Fig. 3, in each second braid wire 12, an axial distance L between any two adjacent active coils of the opaque filament 122 is 1.2 times a diameter of the opaque filament 122.

The first braid wires 11 have a diameter of 45 µm, and the second braid wires 12 have a diameter of 85 µm. The tubular structure can be collapsed to have a first diameter of 0.029 inches for delivery. The number of the first braid wires 11 is 44 and the number of the second braid wires 12 is 4. In an expanded configuration of the self-expanding braided tubular structure, the first braid wires 11 and the second braid wires 12 are evenly spaced circumferentially. The first braid wires 11 and the second braid wires 12 are surface-coated with a drug coating.

In the expanded configuration, the self-expanding braided tubular structure includes a first section 22, an intermediate section 21 and a second section 23, which are sequentially joined together. In the self-expanding braided tubular structure, the intermediate section 21 maintains a constant outer diameter, while each of the first section 22 and the second section 23 has an outer diameter gradually increasing from the intermediate section 21 toward a corresponding end opening of the self-expanding braided tubular structure. Thus, the outer diameters d2 of the first section 22 and the second section 23 at the end openings are greater than the diameter d1 of the intermediate section 21. The first section 22 and the second section 23 may be both flared in shape, as shown in Fig. 1. The first section 22 and the second section 23 both have an axial length of 0.5 mm. The first section 22 and the second section 23 both form an angle α of 30° with respect to a center axis of the self-expanding braided tubular structure.

### Embodiment 2

Referring to Figs. 1, 2 and 3, a vascular stent according to an embodiment is provided in the form of a self-expanding braided tubular structure constructed from interwoven spiral first and second components. The first components are first braid wires 11, and the second components are composite braid wires 1 consisting of combined first braid wires 11 and second braid wires 12. Each first braid wire 11 includes a first wire core 111 and a cladding jacket 112 covering the first wire core 111. The first wire core 111 is made of platinum, and the cladding jacket 112 is made of a cobalt-chromium alloy. Each second braid wire 12 includes a second wire core 121 and an opaque filament 122 wound on the second wire core 121. The opaque filament 122 is a platinum filament, and the second wire core 121 is provided by one first braid wire 11. In the first braid wires 11, the first wire cores 111 have a linear attenuation coefficient of 5148 cm⁻¹, and the cladding jackets 112 have a linear attenuation coefficient of 379 cm⁻¹. In the second braid wires 12, the opaque filaments 122 have a linear attenuation coefficient of 5148 cm⁻¹, and the second wire cores 121 have a linear attenuation coefficient of 952 cm⁻¹. Among the four materials from which the self-expanding braided tubular structure is fabricated, the ratio of the highest linear attenuation coefficient to the lowest linear attenuation coefficient is about 14.

The second braid wires 12 have a circular cross-section. Referring to Fig. 3, in each second braid wire 12, an axial distance L between any two adjacent active coils of the opaque filament 122 is 1.1 times a diameter of the opaque filament 122.

The first braid wires 11 have a diameter of 40 µm, and the second braid wires 12 have a diameter of 75 µm. The tubular structure can be collapsed to have a first diameter of 0.027 inches for delivery. The number of the first braid wires 11 is 46 and the number of the second braid wires 12 is 2. In an expanded configuration of the self-expanding braided tubular structure, the first braid wires 11 and the second braid wires 12 are evenly spaced circumferentially.

The first braid wires 11 and the second braid wires 12 are surface-coated with an antithrombotic coating, which imparts improved antithrombotic properties to the vascular stent.

In the expanded configuration, the self-expanding braided tubular structure includes a first section 22, an intermediate section 21 and a second section 23, which are sequentially joined together. In the self-expanding braided tubular structure, the intermediate section 21 maintains a constant outer diameter, while each of the first section 22 and the second section 23 has an outer diameter gradually increasing from the intermediate section 21 toward a corresponding end opening of the self-expanding braided tubular structure. Thus, the outer diameters d2 of the first section 22 and the second section 23 at the end openings are greater than the diameter d1 of the intermediate section 21. The first section 22 and the second section 23 may be both flared in shape, as shown in Fig. 1. The first section 22 and the second section 23 both have an axial length of 0.5 mm. The first section 22 and the second section 23 both form an angle α of 45° with respect to a center axis of the self-expanding braided tubular structure.

### Embodiment 3

Referring to Figs. 1, 2 and 3, a vascular stent according to an embodiment is provided in the form of a self-expanding braided tubular structure constructed from interwoven spiral first and second components. The first components are first braid wires 11, and the second components are composite braid wires 1 consisting of combined first braid wires 11 and second braid wires 12. Each first braid wire 11 includes a first wire core 111 and a cladding jacket 112 covering the first wire core 111. The first wire core 111 is made of tantalum, and the cladding jacket 112 is made of a cobalt-chromium alloy. Each second braid wire 12 includes a second wire core 121 and an opaque filament 122 wound on the second wire core 121. The opaque filament 122 is a tantalum filament, and the second wire core 121 is provided by one first braid wire 11. In the first braid wires, the first wire cores 111 have a linear attenuation coefficient of 1671 cm⁻¹, and the cladding jackets 112 have a linear attenuation coefficient of 379 cm⁻¹. In the second braid wires 12, the opaque filaments 122 have a linear attenuation coefficient of 1671 cm⁻¹, and the second wire cores 121 have a linear attenuation coefficient of 952 cm⁻¹. Among the four materials from which the self-expanding braided tubular structure is fabricated, the ratio of the highest linear attenuation coefficient to the lowest linear attenuation coefficient is about 4.4.

The second braid wires 12 have a circular cross-section. Referring to Fig. 3, in each second braid wire 12, an axial distance L between any two adjacent active coils of the opaque filament 122 is 1.0 times a diameter of the opaque filament 122.

The first braid wires 11 have a diameter of 35 µm, and the second braid wires 12 have a diameter of 50 µm. The tubular structure can be collapsed to have a first diameter of 0.021 inches for delivery.

The number of the first braid wires 11 is 60 and the number of the second braid wires 12 is 4. In an expanded configuration of the self-expanding braided tubular structure, the first braid wires 11 and the second braid wires 12 are evenly spaced circumferentially.

The first braid wires 11 and the second braid wires 12 are surface-coated by dipping with a hydrophilic coating.

In the expanded configuration, the self-expanding braided tubular structure includes a first section 22, an intermediate section 21 and a second section 23, which are sequentially joined together. In the self-expanding braided tubular structure, the intermediate section 21 maintains a constant outer diameter, while each of the first section 22 and the second section 23 has an outer diameter gradually increasing from the intermediate section 21 toward a corresponding end opening of the self-expanding braided tubular structure. Thus, the outer diameters d2 of the first section 22 and the second section 23 at the end openings are greater than the diameter d1 of the intermediate section 21. The first section 22 and the second section 23 may be both flared in shape, as shown in Fig. 1. The first section 22 and the second section 23 both have an axial length of 1.0 mm. The first section 22 and the second section 23 both form an angle α of 60° with respect to a center axis of the self-expanding braided tubular structure.

### Embodiment 4

Referring to Figs. 1, 2 and 3, a vascular stent according to an embodiment is provided in the form of a self-expanding braided tubular structure constructed from interwoven spiral first and second components. The first components are first braid wires 11, and the second components are composite braid wires 1 consisting of combined first braid wires 11 and second braid wires 12. Each first braid wire 11 includes a first wire core 111 and a cladding jacket 112 covering the first wire core 111. The first wire core 111 is made of gold, and the cladding jacket 112 is made of a cobalt-chromium alloy. Each second braid wire 12 includes a second wire core 121 and an opaque filament 122 wound on the second wire core 121. The opaque filament 122 is a tantalum filament, and the second wire core 121 is provided by one first braid wire 11. In the first braid wires, the first wire cores 111 have a linear attenuation coefficient of 3864 cm⁻¹, and the cladding jackets 112 have a linear attenuation coefficient of 379 cm⁻¹. In the second braid wires 12, the opaque filaments 122 have a linear attenuation coefficient of 1671 cm⁻¹, and the second wire cores 121 have a linear attenuation coefficient of 952 cm⁻¹. Among the four materials from which the self-expanding braided tubular structure is fabricated, the ratio of the highest linear attenuation coefficient to the lowest linear attenuation coefficient is about 10.2.

The second braid wires 12 have a circular cross-section. Referring to Fig. 3, in each second braid wire 12, an axial distance L between any two adjacent active coils of the opaque filament 122 is 1.5 times a diameter of the opaque filament 122.

Additionally, the first braid wires 11 have a diameter of 25 µm, and the second braid wires 12 have a diameter of 25 µm. The tubular structure can be collapsed to have a first diameter and delivered by a 0.017-inch catheter to the site of an aneurysm.

The number of the first braid wires 11 is 62 and the number of the second braid wires 12 is 2. In an expanded configuration of the self-expanding braided tubular structure, the first braid wires 11 and the second braid wires 12 are evenly spaced circumferentially.

Further, the first braid wires 11 and the second braid wires 12 are surface-coated by dipping with a drug coating.

In the expanded configuration, the self-expanding braided tubular structure includes a first section 22, an intermediate section 21 and a second section 23, which are sequentially joined together. In the self-expanding braided tubular structure, the intermediate section 21 maintains a constant outer diameter, while each of the first section 22 and the second section 23 has an outer diameter gradually increasing from the intermediate section 21 toward a corresponding end opening of the self-expanding braided tubular structure. Thus, the outer diameters d2 of the first section 22 and the second section 23 at the end openings are greater than the diameter d1 of the intermediate section 21. The first section 22 and the second section 23 may be both flared in shape, as shown in Fig. 1. The first section 22 and the second section 23 both have an axial length of 2 mm. The first section 22 and the second section 23 both form an angle α of 35° with respect to a center axis of the self-expanding braided tubular structure.

### Comparative Embodiment

A vascular stent product A from a foreign medical device manufacturer is a stent braided from 36 MP35N (a nickel-cobalt-chromium-molybdenum alloy) wires and 12 platinum wires, all having a diameter of 30 µm. The MP35N material has a density of 8.41g/cm³, and platinum has a density of 21.45g/cm³. At a wavelength of 0.07107 nm, the MP35N material shows a mass absorption coefficient of about 35-45 cm²/g, and platinum exhibits a mass absorption coefficient of 200-240 cm²/g. For example, assume that the vascular stent in Embodiment 2 is braided only with the spiral first braid wires 11, which have a diameter of 40 µm, a density of 13.6 g/cm³ and a mass absorption coefficient of 60-70 cm²/g. According to the intensity attenuation formula: I=I₀e^{-µt} (where, t denotes a thickness of a uniform material, corresponding to the diameters of the braid wires in the context of the present invention), when an X-ray beam with an intensity of I₀ radiated to the material having the thickness of t transmits through the material, its intensity will be attenuated to I. A smaller I value will indicate greater attenuation by and better fluoroscopic visibility of the material. In the vascular stent product A, I=I₀e^{-1.01} for MP35N, and I=I₀e^{-14.2} for platinum. For the first braid wires 11 in Embodiment 2, I=I₀e^{-3.5}. Thus, the fluoroscopic visibility of the first braid wires 11 in Embodiment 2 is better than that of the MP35N wires but slightly worse than that of the platinum wires. Therefore, if the vascular stent in Embodiment 2 were braided only with the first braid wires, compared with the conventional product, although its overall fluoroscopic visibility would have been better, the benefit would have been insignificant. In the second braid wires additionally included in Embodiment 2, the second wire cores 121 have a diameter of 35 µm, a density of 6.45 g/cm³ and a mass absorption coefficient of 30-40 cm²/g, and the opaque filaments 122 have a density 21.45 g/cm³ and a mass absorption coefficient of the 200-240 cm²/g. Thus, for the second braid wire 12, I=I₀e^{-24.24}. Obviously, the fluoroscopic visibility of the second braid wires 12 is superior over that of the platinum wires in the vascular stent product A. As a result, the fluoroscopic visibility of the vascular stent in Embodiment 2 is better than that of the vascular stent product A. Moreover, the second braid wires 12 are more fluoroscopically visible under X-ray than other braid wires and are arranged on circumferentially opposing locations. Thus, it can be determined whether there is inward warpage at an end of the expanded vascular stent simply by checking a diameter at the end through measuring the distance between the circumferential locations.

Therefore, the vascular stents provided in the present invention have at least the following advantages:
1. As each braid wire in each vascular stent contains both a radiopaque material and an elastic material, both the whole vascular stent and each braid wire are fluoroscopically visible and have shape memory properties, enabling self-expansion to a predetermined size.
2. In the vascular stents, the first braid wires, each consisting of an elastic outer cladding jacket and a radiopaque inner first wire core, are braided as the main part. The elastic material, which accounts for a sufficiently large proportion, provides drive for self-expansion and strong support. The opaque wire cores are visible on DSA such that boundaries of the vascular stent can be clearly identified during a surgical procedure and a physician can clearly determine whether the vascular stent has adhered to the wall of an intended blood vessel.
3. The second braid wires added contain a material with even greater radiopaqueness and are thus more fluoroscopically visible under X-ray than other braid wires. Moreover, they are arranged at circumferentially opposing locations. In this way, it can be determined whether there is inward warpage at a leading end of the expanded vascular stent simply by checking a diameter at the end through measuring the distance(s) between the circumferential locations.

While the present invention has been described above in terms of preferred embodiments, it is not limited to the embodiments disclosed herein. Any person of skill in the art can make changes and modifications without departing from the scope of the invention as defined by the appended claims.

## Claims

1. A vascular stent, wherein the vascular stent is a self-expanding braided tubular structure constructed from at least one first braid wire (11) and a composite braid wire (1), which are interwoven with each other, wherein:
each first braid wire (11) comprises a first wire core (111) and a cladding jacket (112) covering the first wire core (111); and
the composite braid wire (1) consists of the at least one first braid wire (11) and at least one second braid wire (12), wherein
each second braid wire (12) comprises a second wire core (121) and **characterised in that** an opaque filament (122) is wound on the second wire core (121).

2. The vascular stent of claim 1, wherein the vascular stent is a self-expanding braided tubular structure constructed from the at least one first braid wire (11) and the composite braid wire (1), which are spiral and interwoven with each other.

3. The vascular stent of claim 2, wherein the vascular stent is a self-expanding braided tubular structure constructed from the at least one first braid wire (11) and the composite braid wire (1), which are interwoven with each other so as to spiral respectively clockwise and counterclockwise from opposing ends of the vascular stent.

4. The vascular stent of claim 1, wherein the cladding jacket (112) is made of an elastic biomaterial and the first wire core (111) is made of a radiopaque material, the material of the first wire core (111) having a linear attenuation coefficient higher than a linear attenuation coefficient of the material of the cladding jacket (112).

5. The vascular stent of claim 4, wherein the radiopaque material is one of platinum, iridium, gold, silver and tantalum, or an alloy thereof and wherein the elastic biomaterial is one or more of a nickel-titanium alloy, nitinol, stainless steel, a cobalt-chromium alloy and a nickel-cobalt alloy.

6. The vascular stent of claim 1, wherein the first braid wire (11) has a cross-section shape of circular, square, elliptic or trapezoidal.

7. The vascular stent of claim 6, wherein a cross-section area of the first wire core (111) accounts for 10-40% of a total cross-section area of the first braid wire (11).

8. The vascular stent of claim 1, wherein among all the materials in the first braid wire (11) and the second braid wire (12), a greatest linear attenuation coefficient does not exceed 25 times a lowest linear attenuation coefficient.

9. The vascular stent of claim 8, wherein a linear attenuation coefficient of the opaque filament (122) does not exceed 25 times of a linear attenuation coefficient of the first wire core (111).

10. The vascular stent of claim 1, wherein the second braid wire (12) has a cross-section shape of circular, square, elliptic or trapezoidal.

11. The vascular stent of claim 1, wherein the opaque filament (122) is one of platinum, iridium, gold, silver and tantalum, or an alloy thereof and wherein the second wire core (121) is a nickel-titanium alloy or one first braid wire.

12. The vascular stent of claim 5, wherein an axial distance between any two adjacent coils of the opaque filament (122) wound on the second wire core (121) is 1.0-1.5 times a diameter of the opaque filament (122).

13. The vascular stent of claim 1, wherein a number of the first braid wires (11) is 24-96 and a number of the second braid wires (12) is 2-6.

14. The vascular stent of claim 1, wherein the first braid wire (11) and/or the second braid wire (12) is/are surface coated by spraying or dipping with a drug coating, an antithrombotic coating and/or a hydrophilic coating.

15. The vascular stent of claim 1, wherein the self-expanding braided tubular structure has a first diameter less than 0.74 mm in a collapsed configuration and a second diameter in the range of 1.5 mm to 7 mm in an expanded configuration.

16. The vascular stent of claim 15, wherein the self-expanding braided tubular structure expands from the first diameter to the second diameter under the action of a force in the range of 0.01 N to 1 N.

17. The vascular stent of claim 15, wherein each first braid wire (11) has a cross-section diameter not greater than 50 µm and wherein each second braid wire (12) has a cross-section diameter not greater than 90 µm.

18. The vascular stent of claim 17, wherein:
(i) the first diameter of the self-expanding braided tubular structure in the collapsed configuration is less than 0.6858 mm, wherein the cross-section diameter of each second braid wire (12) is not greater than 80 µm and the cross-section diameter of each first braid wire (11) is not greater than 45 µm; or
(ii) wherein the first diameter of the self-expanding braided tubular structure in the collapsed configuration is less than 0.5334 mm, wherein the cross-section diameter of each second braid wire (12) is not greater than 50 µm and the cross-section diameter of each first braid wire (11) is not greater than 35 µm; or
(iii) wherein the first diameter of the self-expanding braided tubular structure in the collapsed configuration is less than 0.4826 mm, wherein the cross-section diameter of each second braid wire (12) is not greater than 30 µm and the cross-section diameter of each first braid wire (11) is not greater than 30 µm.

19. The vascular stent of claim 1, wherein in the expanded configuration, the self-expanding braided tubular structure has a first section (22), an intermediate section (21) and a second section (23), which are sequentially joined together, the intermediate section (21) of the self-expanding braided tubular structure having a constant outer diameter, each of the first section (22) and the second section (23) of the self-expanding braided tubular structure having an outer diameter gradually increasing from the intermediate section (21) toward a corresponding end opening of the self-expanding braided tubular structure.

20. The vascular stent of claim 19, wherein the intermediate section (21) has an axial length of from 2 mm to 60 mm, wherein each of the first section (22) and the second section (23) has an axial length of from 0.5 mm to 3 mm, and wherein wire segments in the first section (22) or the second section (23), the outer diameter of which gradually increases from the intermediate section (21) toward the corresponding end opening of the self-expanding braided tubular structure extend at an angle of 10-60° with respect to a center axis of the self-expanding braided tubular structure.

21. The vascular stent of claim 19, wherein the first section (22) and the second section (23) of the self-expanding braided tubular structure are flared in shape, in the shape of truncated cones, or tapered, or the first section (22) and the second section (23) have cross-sections of star shape.

22. The vascular stent of claim 1, wherein the self-expanding braided tubular structure has an axial length of 2-70 mm and wherein the interwoven two sets of braid wires form an angle of 100-140° axially and form an angle of 40-80° circumferentially.

## Patentansprüche

1. Gefäßstent, wobei der Gefäßstent eine selbstexpandierende, geflochtene Röhrenstruktur ist, die aus mindestens einem ersten Geflechtdraht (11) und einem Verbundgeflechtdraht (1) konstruiert ist, die miteinander verflochten sind, wobei:
jeder erste Geflechtdraht (11) einen ersten Drahtkern (111) und einen den ersten Drahtkern (111) bedeckenden Verkleidungsmantel (112) umfasst; und
der Verbundgeflechtdraht (1) aus dem mindestens einen ersten Geflechtdraht (11) und mindestens einem zweiten Geflechtdraht (12) besteht, wobei
jeder zweite Geflechtdraht (12) einen zweiten Drahtkern (121) umfasst, und **dadurch gekennzeichnet, dass** auf dem zweiten Drahtkern (121) ein undurchsichtiger Faden (122) gewickelt ist.

2. Gefäßstent nach Anspruch 1, wobei der Gefäßstent eine selbstexpandierende, geflochtene Röhrenstruktur ist, die aus dem mindestens einen ersten Geflechtdraht (11) und dem Verbundgeflechtdraht (1) konstruiert ist, die spiralförmig und miteinander verflochten sind.

3. Gefäßstent nach Anspruch 2, wobei der Gefäßstent eine selbstexpandierende, geflochtene Röhrenstruktur ist, die aus dem mindestens einem ersten Geflechtdraht (11) und dem Verbundgeflechtdraht (1) konstruiert ist, die miteinander verflochten sind, um sich von gegenüberliegenden Enden des Gefäßstents jeweils im Uhrzeigersinn und gegen den Uhrzeigersinn spiralförmig zu winden.

4. Gefäßstent nach Anspruch 1, wobei der Verkleidungsmantel (112) aus einem elastischen Biomaterial hergestellt ist und der erste Drahtkern (111) aus einem röntgendichten Material hergestellt ist, wobei das Material des ersten Drahtkerns (111) einen linearen Schwächungskoeffizienten aufweist, der höher ist als einer lineare Schwächungskoeffizient des Materials des Verkleidungsmantels (112).

5. Gefäßstent nach Anspruch 4, wobei das röntgendichte Material eins von Platin, Iridium, Gold, Silber und Tantal oder einer Legierung davon ist und wobei das elastische Biomaterial eins oder mehrere von einer Nickel-Titan-Legierung, Nitinol, Edelstahl, einer Kobalt-Chrom-Legierung und einer Nickel-Kobalt-Legierung ist.

6. Gefäßstent nach Anspruch 1, wobei der erste Geflechtdraht (11) eine kreisförmige, quadratische, elliptische oder trapezförmige Querschnittsgestalt aufweist.

7. Gefäßstent nach Anspruch 6, wobei eine Querschnittsfläche des ersten Drahtkerns (111) 10-40 % einer gesamten Querschnittsfläche des ersten Geflechtdrahtes (11) ausmacht.

8. Gefäßstent nach Anspruch 1, wobei unter allen Materialien in dem ersten Geflechtdraht (11) und dem zweiten Geflechtdraht (12) ein größter linearer Schwächungskoeffizient nicht das 25-fache eines geringsten linearen Schwächungskoeffizienten überschreitet.

9. Gefäßstent nach Anspruch 8, wobei ein linearer Schwächungskoeffizient des undurchsichtigen Fadens (122) nicht das 25-fache eines linearen Schwächungskoeffizienten des ersten Drahtkerns (111) überschreitet.

10. Gefäßstent nach Anspruch 1, wobei der zweite Geflechtdraht (12) eine kreisförmige, quadratische, elliptische oder trapezförmige Querschnittsgestalt aufweist.

11. Gefäßstent nach Anspruch 1, wobei der undurchsichtige Faden (122) einer aus Platin, Iridium, Gold, Silber und Tantal oder einer Legierung davon ist und wobei der zweite Drahtkern (121) eine Nickel-Titan-Legierung oder ein erster Geflechtdraht ist.

12. Gefäßstent nach Anspruch 5, wobei ein axialer Abstand zwischen beliebigen zwei benachbarten Windungen des undurchsichtigen Fadens (122), der auf dem zweiten Drahtkern (121) gewickelt ist, das 1,0- bis 1,5-fache eines Durchmessers des undurchsichtigen Fadens (122) beträgt.

13. Gefäßstent nach Anspruch 1, wobei eine Anzahl der ersten Geflechtdrähte (11) 24-96 beträgt und eine Anzahl der zweiten Geflechtdrähte (12) 2-6 beträgt.

14. Gefäßstent nach Anspruch 1, wobei der erste Geflechtdraht (11) und/oder der zweite Geflechtdraht (12) durch Besprühen oder Eintauchen mit einer Arzneimittelbeschichtung, einer antithrombotischen Beschichtung und/oder einer hydrophilen Beschichtung oberflächenbeschichtet ist/sind.

15. Gefäßstent nach Anspruch 1, wobei die selbstexpandierende, geflochtene Röhrenstruktur in einer zusammengefalteten Konfiguration einen ersten Durchmesser von weniger als 0,74 mm und in einer expandierten Konfiguration einen zweiten Durchmesser im Bereich von 1,5 mm bis 7 mm aufweist.

16. Gefäßstent nach Anspruch 15, wobei die selbstexpandierende, geflochtene Röhrenstruktur unter der Einwirkung einer Kraft im Bereich von 0,01 N bis 1 N von dem ersten Durchmesser zu dem zweiten Durchmesser expandiert.

17. Gefäßstent nach Anspruch 15, wobei jeder erste Geflechtdraht (11) einen Querschnittsdurchmesser von nicht mehr als 50 µm aufweist und jeder zweite Geflechtdraht (12) einen Querschnittsdurchmesser von nicht mehr als 90 µm aufweist.

18. Gefäßstent nach Anspruch 17, wobei:
(i) der erste Durchmesser der selbstexpandierenden, geflochtenen Röhrenstruktur in der zusammengefalteten Konfiguration weniger als 0,6858 mm beträgt, wobei der Querschnittsdurchmesser jedes zweiten Geflechtdrahtes (12) nicht mehr als 80 µm beträgt und der Querschnittsdurchmesser jedes ersten Geflechtdrahtes (11) nicht mehr als 45 µm beträgt; oder
(ii) wobei der erste Durchmesser der selbstexpandierenden, geflochtenen Röhrenstruktur in der zusammengefalteten Konfiguration weniger als 0,5334 mm beträgt, wobei der Querschnittsdurchmesser jedes zweiten Geflechtdrahtes (12) nicht mehr als 50 µm beträgt und der Querschnittsdurchmesser jedes ersten Geflechtdrahtes (11) nicht mehr als 35 µm beträgt; oder
(iii) wobei der erste Durchmesser der selbstexpandierenden, geflochtenen Röhrenstruktur in der zusammengefalteten Konfiguration weniger als 0,4826 mm beträgt, wobei der Querschnittsdurchmesser jedes zweiten Geflechtdrahtes (12) nicht mehr als 30 µm beträgt und der Querschnittsdurchmesser jedes ersten Geflechtdrahtes (11) nicht mehr als 30 µm beträgt.

19. Gefäßstent nach Anspruch 1, wobei die selbstexpandierende, geflochtene Röhrenstruktur in der expandierten Konfiguration einen ersten Abschnitt (22), einen mittleren Abschnitt (21) und einen zweiten Abschnitt (23) aufweist, die nacheinander zusammen verbunden sind, wobei der mittlere Abschnitt (21) der selbstexpandierenden, geflochtenen Röhrenstruktur einen konstanten Außendurchmesser aufweist, wobei jeder des ersten Abschnitts (22) und des zweiten Abschnitts (23) der selbstexpandierenden, geflochtenen Röhrenstruktur einen Außendurchmesser aufweist, der von dem mittleren Abschnitt (21) hin zu einer entsprechenden Endöffnung der selbstexpandierenden, geflochtenen Röhrenstruktur allmählich zunimmt.

20. Gefäßstent nach Anspruch 19, wobei der mittlere Abschnitt (21) eine axiale Länge von 2 mm bis 60 mm aufweist, wobei jeder des ersten Abschnitts (22) und des zweiten Abschnitts (23) eine axiale Länge von 0,5 mm bis 3 mm aufweist und wobei Drahtsegmente in dem ersten Abschnitt (22) oder dem zweiten Abschnitt (23), deren Außendurchmesser von dem mittleren Abschnitt (21) hin zu der entsprechenden Endöffnung der selbstexpandierenden, geflochtenen Röhrenstruktur allmählich zunimmt, sich bezüglich einer Mittelachse der selbstexpandierenden, geflochtenen Röhrenstruktur in einem Winkel von 10-60° erstrecken.

21. Gefäßstent nach Anspruch 19, wobei der erste Abschnitt (22) und der zweite Abschnitt (23) der selbstexpandierenden, geflochtenen Röhrenstruktur in der Gestalt aufgeweitet, in der Gestalt von Kegelstümpfen oder sich verjüngend vorliegen oder der erste Abschnitt (22) und der zweite Abschnitt (23) Querschnitte in Sterngestalt aufweisen.

22. Gefäßstent nach Anspruch 1, wobei die selbstexpandierende, geflochtene Röhrenstruktur eine axiale Länge von 2-70 mm aufweist und wobei die verflochtenen zwei Geflechtdrahtsätze axial einen Winkel von 100-140° bilden und in Umfangsrichtung einen Winkel von 40-80° bilden.

## Revendications

1. Stent vasculaire, dans lequel le stent vasculaire est une structure tubulaire tressée auto-expansible construite à partir d'au moins un premier fil tressé (11) et d'un fil tressé composite (1), qui sont entrelacés l'un avec l'autre, dans lequel :
chaque premier fil de tresse (11) comprend un premier noyau (111) et une gaine de revêtement (112) recouvrant le premier noyau (111) ; et
le fil tressé composite (1) est constitué du au moins un premier fil tressé (11) et d'au moins un deuxième fil tressé (12), dans lequel
chaque deuxième fil de tresse (12) comprend un deuxième noyau de fil (121) et est **caractérisé en ce qu'**un filament opaque (122) est enroulé sur le deuxième noyau de fil (121).

2. Stent vasculaire selon la revendication 1, dans lequel le stent vasculaire est une structure tubulaire tressée auto-expansible construite à partir d'au moins un premier fil tressé (11) et du fil tressé composite (1), qui sont spiralés et entrelacés les uns avec les autres.

3. Stent vasculaire selon la revendication 2, dans lequel le stent vasculaire est une structure tubulaire tressée auto-expansible construite à partir de l'au moins un premier fil tressé (11) et du fil tressé composite (1), qui sont entrelacés l'un avec l'autre de manière à s'enrouler respectivement dans le sens horaire et antihoraire à partir des extrémités opposées du stent vasculaire.

4. Stent vasculaire selon la revendication 1, dans lequel la gaine de revêtement (112) est réalisée en un biomatériau élastique et le premier noyau de fil (111) est fait d'un matériau radio-opaque, le matériau du premier noyau de fil (111) présentant un coefficient d'atténuation linéaire supérieur à un coefficient d'atténuation linéaire du matériau de la gaine de revêtement (112).

5. Stent vasculaire selon la revendication 4, dans lequel le matériau radio-opaque est du platine, de l'iridium, de l'or, de l'argent et du tantale, ou un alliage de ceux-ci et dans lequel le biomatériau élastique est un ou plusieurs parmi un alliage nickel-titane, du nitinol, de l'acier inoxydable, un alliage cobalt-chrome et un alliage nickel-cobalt.

6. Stent vasculaire selon la revendication 1, dans lequel le premier fil tressé (11) présente une forme en coupe transversale circulaire, carrée, elliptique ou trapézoïdale.

7. Stent vasculaire selon la revendication 6, dans lequel une surface de section transversale du premier noyau de fil (111) représente 10 à 40 % d'une surface de section transversale totale du premier fil tressé (11).

8. Stent vasculaire selon la revendication 1, dans lequel, parmi tous les matériaux du premier fil tressé (11) et du deuxième fil tressé (12), un coefficient d'atténuation linéaire maximal ne dépasse pas 25 fois un coefficient d'atténuation linéaire minimal.

9. Stent vasculaire selon la revendication 8, dans lequel un coefficient d'atténuation linéaire du filament opaque (122) ne dépasse pas 25 fois un coefficient d'atténuation linéaire du premier noyau de fil (111).

10. Stent vasculaire selon la revendication 1, dans lequel le deuxième fil tressé (12) présente une forme en coupe transversale circulaire, carrée, elliptique ou trapézoïdale.

11. Stent vasculaire selon la revendication 1, dans lequel le filament opaque (122) est en platine, en iridium, en or, en argent ou en tantale, ou en un alliage de ceux-ci et dans lequel le deuxième noyau de fil (121) est un alliage de nickel-titane ou un premier fil tressé.

12. Stent vasculaire selon la revendication 5, dans lequel une distance axiale entre deux spires adjacentes quelconques du filament opaque (122) enroulé sur le deuxième noyau de fil (121) est de 1,0 à 1,5 fois le diamètre du filament opaque (122).

13. Stent vasculaire selon la revendication 1, dans lequel un nombre de fils de la première tresse (11) est de 24 à 96 et un nombre de fils de la deuxième tresse (12) est de 2 à 6.

14. Stent vasculaire selon la revendication 1, dans lequel le premier fil tressé (11) et/ou le deuxième fil tressé (12) est/sont revêtu(s) en surface par pulvérisation ou trempage dans un revêtement médicamenteux, un revêtement antithrombotique et/ou un revêtement hydrophile.

15. Stent vasculaire selon la revendication 1, dans lequel la structure tubulaire tressée auto-expansible présente un premier diamètre inférieur à 0,74 mm en configuration repliée et un deuxième diamètre compris dans la plage de 1,5 mm et 7 mm en configuration déployée.

16. Stent vasculaire selon la revendication 15, dans lequel la structure tubulaire tressée auto-expansible s'étend du premier diamètre au deuxième diamètre sous l'action d'une force comprise dans la plage 0,01 N et 1 N.

17. Stent vasculaire selon la revendication 15, dans lequel chaque premier fil tressé (11) présente un diamètre de section transversale ne dépassant pas 50 µm et dans lequel chaque deuxième fil tressé (12) présente un diamètre de section transversale ne dépassant pas 90 µm.

18. Stent vasculaire selon la revendication 17, dans lequel :
(i) le premier diamètre de la structure tubulaire tressée auto-expansible en configuration repliée est inférieur à 0,6858 mm, dans lequel le diamètre de la section transversale de chaque deuxième fil tressé (12) ne dépasse pas 80 µm et le diamètre de la section transversale de chaque premier fil tressé (11) ne dépasse pas 45 µm ; ou
(ii) dans lequel le premier diamètre de la structure tubulaire tressée auto-expansible en configuration repliée est inférieur à 0,5334 mm, dans lequel le diamètre de la section transversale de chaque deuxième fil tressé (12) n'est pas supérieur à 50 µm et le diamètre de la section transversale de chaque premier fil tressé (11) n'est pas supérieur à 35 µm ; ou
(iii) dans lequel le premier diamètre de la structure tubulaire tressée auto-extensible en configuration repliée est inférieur à 0,4826 mm, dans lequel le diamètre de la section transversale de chaque deuxième fil tressé (12) n'est pas supérieur à 30 µm et le diamètre de la section transversale de chaque premier fil tressé (11) n'est pas supérieur à 30 µm.

19. Stent vasculaire selon la revendication 1, dans lequel, dans la configuration déployée, la structure tubulaire tressée auto-expansible comporte une première section (22), une section intermédiaire (21) et une deuxième section (23), qui sont jointes séquentiellement, la section intermédiaire (21) de la structure tubulaire tressée auto-expansible présentant un diamètre extérieur constant, chacune des première (22) et deuxième (23) section de la structure tubulaire tressée auto-expansible présentant un diamètre extérieur augmentant progressivement de la section intermédiaire (21) vers une ouverture d'extrémité correspondante de la structure tubulaire tressée auto-expansible.

20. Stent vasculaire selon la revendication 19, dans lequel la section intermédiaire (21) présente une longueur axiale de 2 mm à 60 mm, dans lequel chacune des première section (22) et deuxième section (23) présente une longueur axiale de 0,5 mm à 3 mm, et dans lequel les segments de fil de la première section (22) ou de la deuxième section (23), dont le diamètre extérieur augmente progressivement de la section intermédiaire (21) vers l'ouverture d'extrémité correspondante de la structure tubulaire tressée auto-expansible, s'étendent selon un angle de 10 à 60° par rapport à un axe central de la structure tubulaire tressée auto-expansible.

21. Stent vasculaire selon la revendication 19, dans lequel la première section (22) et la deuxième section (23) de la structure tubulaire tressée auto-expansible sont évasées, en forme de cônes tronqués ou effilées, ou la première section (22) et la deuxième section (23) présentent des sections transversales en forme d'étoile.

22. Stent vasculaire selon la revendication 1, dans lequel la structure tubulaire tressée auto-expansible présente une longueur axiale de 2 à 70 mm et dans lequel les deux ensembles de fils tressés entrelacés forment un angle de 100 à 140° axialement et un angle de 40 à 80° circonférentiellement.
